# EUROPEAN PATENT APPLICATION

(11) **EP 4 710 781 A1**
(43) Date of publication of application: **18.03.2026**
(21) Application number: 24806525.2
(22) Date of filing: 11.05.2024
(51) Int. Cl.: A24F 40/20, A24F 40/40, A61M 15/00

(54) **ATOMIZATION MEDIUM ASSEMBLY AND AEROSOL GENERATING APPARATUS**

(30) Priority: 12.05.2023 CN 202310538105
(71) Applicant: Smoore International Holdings Limited, Grand Cayman, KY1-1111 (KY)
(72) Inventor: ZHENG, Wei, Shenzhen, Guangdong 518102 (CN); MING, Zhinan, Shenzhen, Guangdong 518102 (CN); XIONG, Wei, Shenzhen, Guangdong 518102 (CN)
(74) Representative: Winter, Brandl - Partnerschaft mbB
(86) International application number: PCT/CN2024/092725
(87) International publication number: WO 2024/235173

(57) **Abstract**

Provided is an atomization substrate assembly. The atomization substrate assembly includes a housing assembly. The housing assembly is provided with a storage space for storing an unatomized strip-shaped substrate, an atomization cavity for heating and atomizing the strip-shaped substrate to form an aerosol, and an accommodation space for accommodating the atomized strip-shaped substrate. At least one partition is formed between the storage space and the accommodation space, and/or at least one partition is formed between the storage space and the atomization cavity. By arranging at least one partition between the storage space and the accommodation space, and at least one partition between the storage space and the atomization cavity, the storage space is separated from the accommodation space and the atomization cavity. Thus, an aerosol in the atomization cavity can be prevented from permeating the storage space and polluting the strip-shaped substrate stored in the storage space, and a tobacco residue scrap or an odor generated in the accommodation space can be prevented from entering the storage space and causing pollution.

## Description

### TECHNICAL FIELD

The present disclosure relates to the field of atomization, and particularly relates to an atomization substrate assembly and an aerosol generating device

### BACKGROUND

In the related technology, aerosol generating substrates are made into strip-type substrates to be quantitatively and continuously supplied to an atomization assembly. After the strip-type substrates are heated, generated aerosols will diffuse to the surroundings and even permeate unbaked and unheated fresh substrates. In this case, the fresh substrates will be polluted and affected with damp. After being heated, the polluted or damped substrates generally have an adverse effect on the flavor, affecting user experience. Moreover, tobacco residue scraps or odors generated after the strip-type substrates are heated will affect the fresh substrates.

### SUMMARY

In view of this, embodiments of the present disclosure expect to provide an atomization substrate assembly and an aerosol generating device which can relieve substrate pollution and improve user experience.

An embodiment of the present disclosure provides an atomization substrate assembly. The atomization substrate assembly includes:
a housing assembly.

The housing assembly is provided with a storage space for storing an unatomized strip-shaped substrate, an atomization cavity for heating and atomizing the strip-shaped substrate to form an aerosol, and an accommodation space for accommodating the atomized strip-shaped substrate.

At least one partition is formed between the storage space and the accommodation space, and/or at least one partition is formed between the storage space and the atomization cavity.

In some embodiments, the at least one partition includes a first partition portion separating the storage space from the accommodation space.

In some embodiments, the first partition portion includes a first curved subsection and a second curved subsection connected to the first curved subsection.

The first curved subsection forms part of the storage space, and the second curved subsection forms part of the accommodation space.

In some embodiments, the first curved subsection and the second curved subsection are connected to form an S-shaped baffle structure.

In some embodiments, the atomization cavity is provided with a material inlet for the strip-shaped substrate to enter the atomization cavity.

The at least one partition includes a second partition portion arranged near or at the material inlet.

In some embodiments, a first substrate pass-through port for the strip-shaped substrate to pass through is formed on the second partition portion. The first substrate pass-through port is in clearance fit with the strip-shaped substrate.

In some embodiments, in the thickness direction of the strip-shaped substrate, the shape of the section of the first substrate pass-through port is the same as or similar to the shape of the section of the strip-shaped substrate.

In some embodiments, the at least one partition includes a third partition portion arranged between the second partition portion and the storage space.

In some embodiments, a second substrate pass-through port for the strip-shaped substrate to pass through is formed on the third partition portion. The second substrate pass-through port is in clearance fit with the strip-shaped substrate.

In some embodiments, in the thickness direction of the strip-shaped substrate, the shape of the section of the second substrate pass-through port is the same as or similar to the shape of the section of the strip-shaped substrate.

In some embodiments, the material inlet is in clearance fit with the strip-shaped substrate.

In some embodiments, in the thickness direction of the strip-shaped substrate, the shape of the section of the material inlet is the same as or similar to the shape of the section of the strip-shaped substrate.

In some embodiments, the at least one partition includes an isolating cover. The isolating cover covers at least the strip-shaped substrate stored in the storage space.

In some embodiments, the isolating cover includes:
a cover body, where the cover body covers the strip-shaped substrate stored in the storage space; and
an extending portion, where one end of the extending portion is connected to the cover body, the other end of the extending portion extends in the direction of the atomization cavity, and the extending portion covers at least part of the strip-shaped substrate between the storage space and the atomization cavity.

In some embodiments, a channel for the strip-shaped substrate to pass through is formed in the extending portion. The channel is in clearance fit with the strip-shaped substrate.

In some embodiments, in the thickness direction of the strip-shaped substrate, the shape of the section of the channel is the same as or similar to the shape of the section of the strip-shaped substrate.

In some embodiments, the at least one partition includes a partition structure integrally formed on the housing assembly, and/or a partition member that is formed independently and then is mounted to the housing assembly.

An embodiment of the present disclosure further provides an aerosol generating device. The aerosol generating device includes:
a main machine, where the main machine is provided with a mounting space; and
the above atomization substrate assembly, where the atomization substrate assembly is arranged in the mounting space.

The atomization substrate assembly provided in embodiments of the present disclosure has at least beneficial effects as follows: by arranging at least one partition between the storage space and the accommodation space, and at least one partition between the storage space and the atomization cavity, the storage space is separated from the accommodation space and the atomization cavity. Thus, an aerosol in the atomization cavity can be prevented from permeating the storage space and polluting the strip-shaped substrate stored in the storage space, and a tobacco residue scrap or an odor generated in the accommodation space can be prevented from entering the storage space and causing pollution.

### BRIEF DESCRIPTION OF THE DRAWINGS

FIG. 1 is a schematic structural diagram of an aerosol generating device according to Embodiment 1 of the present disclosure;
FIG. 2 is an exploded view of FIG. 1;
FIG. 3 is an exploded view of an atomization substrate assembly shown in FIG. 2;
FIG. 4 is a schematic structural diagram of the aerosol generating device shown in FIG. 1 with an outer cover omitted;
FIG. 5 is a schematic structural diagram of the atomization substrate assembly shown in FIG. 2 with a cover body and a feeding assembly omitted;
FIG. 6 is a schematic structural diagram of an atomization substrate assembly according to Embodiment 2 of the present disclosure;

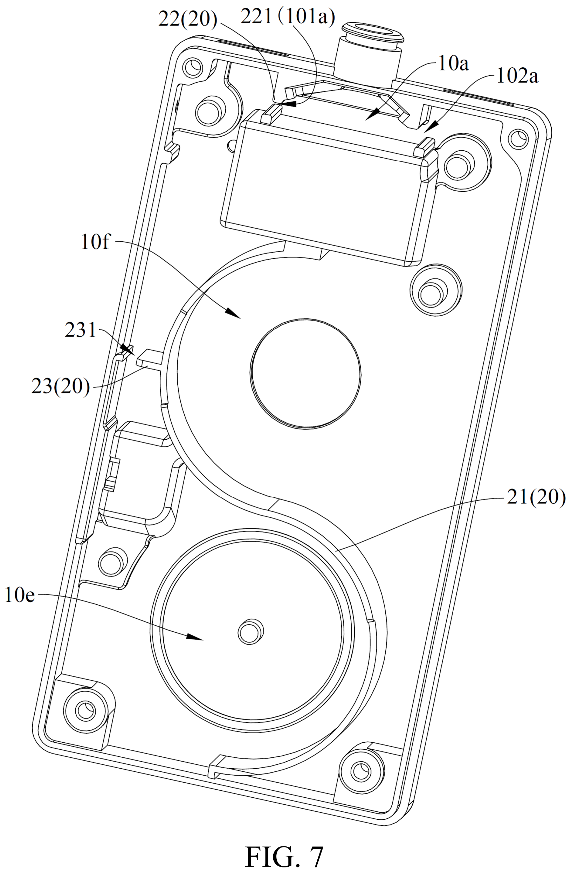

FIG. 7 is a schematic structural diagram of a box body in FIG. 6;

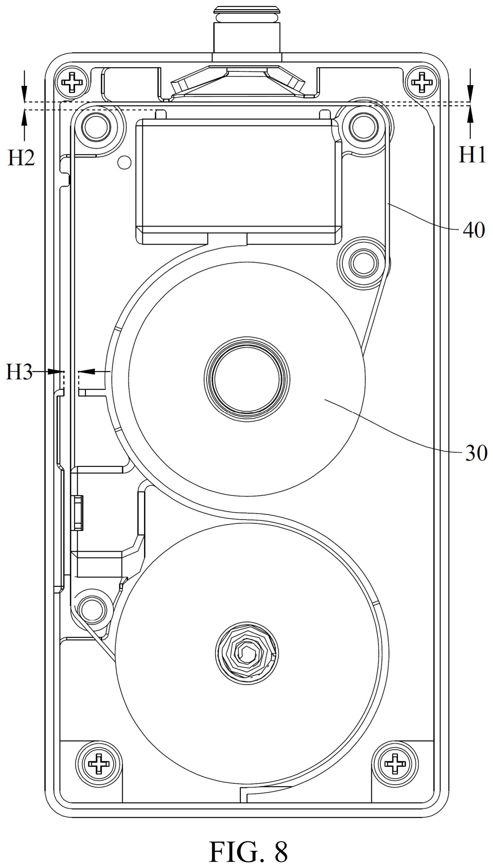

FIG. 8 is a schematic structural diagram of the atomization substrate assembly shown in FIG. 6 with a cover body omitted;
FIG. 9 is a sectional view of the atomization substrate assembly shown in FIG. 6;
FIG. 10 is a schematic structural diagram of the atomization substrate assembly according to Embodiment 3 of the present disclosure with a cover body omitted; and
FIG. 11 is an exploded view of FIG. 10.

### DETAILED DESCRIPTION

The implementations of the present disclosure are further described in detail below in combination with accompanying drawings and embodiments. The following embodiments are used for describing the present disclosure, but cannot be used for limiting the scope of the present disclosure.

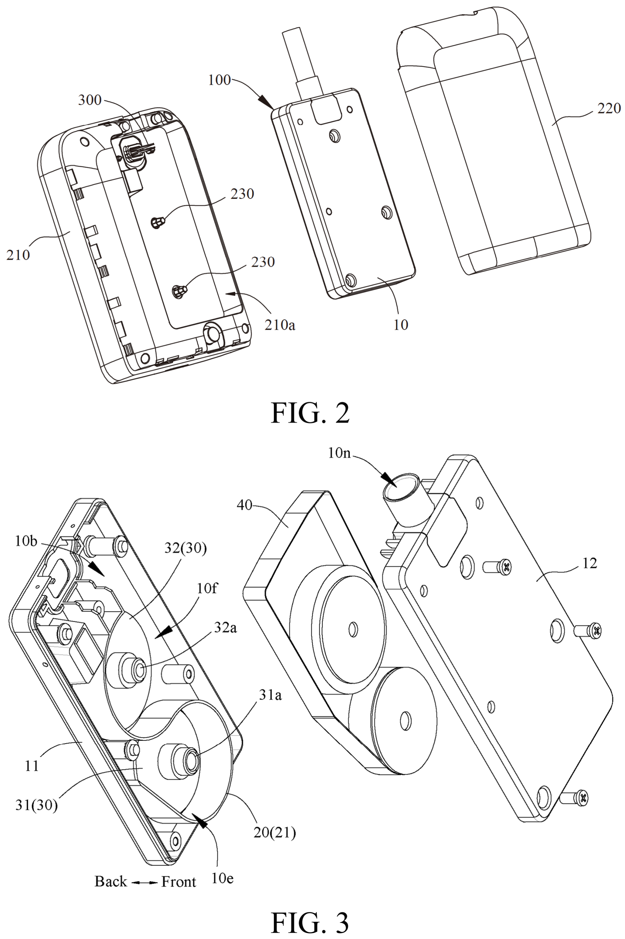

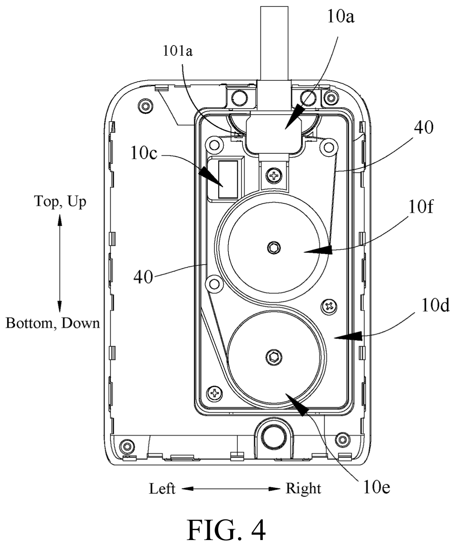

In the descriptions of embodiments of the present disclosure, it should be noted that directional or positional relationships indicated by the terms "up", "down", "front", "back", "left", "right", "top", "bottom", etc. are directional or positional relationships shown based on FIG. 3 and FIG. 4. The term "height direction" refers to the up-down direction, the term "thickness direction" refers to the front-back direction, and the term "width direction" refers to the left-right direction. The terms are merely used for conveniently describing the embodiments of the present disclosure and simplifying the descriptions, but are not used for indicating or implying that an indicated device or element must have a particular direction or be constructed and operated in a particular direction, and thus cannot be understood as limitations on the present disclosure. In addition, the terms "first", "second" and "third" are for descriptive purposes only and should not be construed as indicating or implying relative importance.

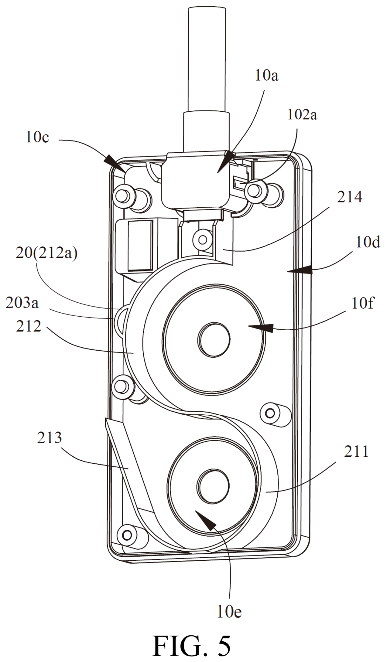

Embodiment 1 of the present disclosure provides an atomization substrate assembly including a strip-shaped substrate. With reference to FIG. 2 to FIG. 5, the atomization substrate assembly 100 may be configured to accommodate a strip-shaped substrates 40.

Embodiment 1 of the present disclosure further provides an aerosol generating device. The atomization substrate assembly includes a strip-shaped substrate 40 and an atomization substrate assembly 100 in any embodiment of the present disclosure.

The atomization substrate assembly 100 includes a housing assembly 10 and a feeding assembly 30.

The housing assembly 10 is provided with a storage space 10e, an accommodation space 10f, and an atomization cavity 10a. The storage space 10e is configured to store the unatomized strip-shaped substrate 40 (a fresh substrate). The accommodation space 10f is configured to accommodate the atomized strip-shaped substrate 40 (an atomized substrate). The atomization cavity 10a is in communication with the outside. The strip-shaped substrate 40 can be atomized in the atomization cavity 10a to generate an aerosol. The aerosol generated in the atomization cavity 10a is discharged for a user to inhale. A method for heating and atomizing the strip-shaped substrate 40 includes but is not limited to one or combinations of a resistance method, an electromagnetic method, a laser method, a microwave method, etc.

The atomization cavity 10a is provided with a material inlet 101a and a material outlet 102a. The strip-shaped substrate 40 can enter the atomization cavity 10a through the material inlet 101a, is heated and atomized in the atomization cavity 10a, and then is transferred out through the material outlet 102a.

After the strip-shaped substrate 40 is heated in the atomization cavity 10a, part of an aerosol generated in the atomization cavity 10a overflows in the direction of the storage space 10e through the material inlet 101a. Part of the aerosol overflows in the direction of the accommodation space 10f through the material outlet 102a. If entering the storage space 10e, the overflowing aerosol will pollute the fresh substrate in the storage space 10e. Thus, the substrate is damped, and changed in flavor and ingredient. Moreover, after the strip-shaped substrate 40 is heated, a tobacco residue scrap or an odor is generated. If the tobacco residue scrap or the odor enters the storage space 10e, the fresh substrate will be affected. Thus, the fresh substrate is required to be separated from the atomized substrate, to be prevented from being polluted and damped.

To resolve the above problem, in the housing assembly 10, at least one partition 20 may be formed between the storage space 10e and the accommodation space 10f, and/or at least one partition may be formed between the storage space and the atomization cavity 10a. Thus, an aerosol overflowing from the atomization cavity 10a can be prevented from entering the storage space 10e and causing pollution, and a tobacco residue scrap or an odor generated in the accommodation space 10f can be prevented from entering the storage space 10e and polluting the fresh substrate. Effectiveness of sealing and storing the fresh substrate is ensured, and a low-cost structural design can be implemented. The partition 20 may include a partition structure integrally formed on the housing assembly 10, and/or a partition member that is formed independently and then is mounted to the housing assembly 10. The partition structure may include one or more of a main rib, a reinforcing rib, a block, a baffle, etc. The partition member may include one or more of an isolating cover, a seal member, a one-way valve, etc.

In some embodiments, at least one partition 20 is formed between the storage space 10e and the accommodation space 10f. On one hand, a tobacco residue scrap or an odor in the accommodation space 10f or a nearby region can be prevented from entering the storage space 10e and causing pollution. On the other hand, an aerosol overflowing from the material outlet 102a of the atomization cavity 10a in the direction of the accommodation space 10f can be prevented from entering the storage space 10e. At least one partition 20 is further formed between the storage space 10e and the atomization cavity 10a. Thus, less or no aerosol overflowing from the material inlet 101a of the atomization cavity 10a in the direction of the storage space 10e enters the storage space 10e.

In some other embodiments, an isolating cover may be directly arranged to cover the fresh substrate in the storage space 10e. The fresh substrate is arranged in a relatively sealed space, to be protected to the greatest extent.

With reference to FIG. 3 to FIG. 5, the at least one partition 20 includes a first partition portion 21 arranged between the storage space 10e and the accommodation space 10f. The housing assembly 10 is provided with an accommodation cavity 10b. The first partition portion 21 partitions the accommodation cavity 10b into a first subcavity 10c and a second subcavity 10d that are independent of each other in the housing assembly 10. That is, through partition by the first partition portion 21, two independent spaces, that is, the first subcavity 10c and the second subcavity 10d are formed in the housing assembly 10.

Illustratively, with reference to FIG. 3, the housing assembly 10 includes a box body 11 and a cover body 12 covering the box body 11. The box body 11 includes an accommodation cavity 10b provided with an opening. The first partition portion 21 is arranged in the accommodation cavity 10b. The cover body 12 covers the opening. The first subcavity 10c and the second subcavity 10d that are independent of each other are defined by the cover body, the box body 11, and the first partition portion 21.

To conveniently assemble or replace the strip-shaped substrate 40, the box body 11 is detachably connected to the cover body 12. That is, when the strip-shaped substrate 40 is required to be assembled or replaced, in a case that the box body 11 is separated from the cover body 12, the cover body 12 is assembled after the strip-shaped substrate 40 is assembled to the box body 11 or replaced. Illustratively, the box body 11 may be connected to the cover body 12, for example, through a magnetic attraction connection, snap-fitting, a threaded connection, plugging, etc. Certainly, in other embodiments, the box body 11 may be non-detachably connected the cover body 12, for example, through an ultrasonic connection or soldering. It should be noted that "non-detachably" means that the box body 11 cannot be detached from the cover body 12 in a case of no damage. The box body 11 and the cover body 12 are used or discarded as a whole.

The feeding assembly 30 is configured to transport the strip-shaped substrate 40 for generating an aerosol. The strip-shaped substrate 40 generates the aerosol in the atomization cavity 10a for user inhalation.

The strip-shaped substrate 40 is arranged in the accommodation cavity 10b and is connected to the feeding assembly 30. The feeding assembly 30 drives the strip-shaped substrate 40 to move in the atomization cavity 10a. Thus, a used strip-shaped substrate 40 in the atomization cavity 10a is moved out of the atomization cavity 10a, and an unused strip-shaped substrate 40 is moved to the atomization cavity 10a to be heated and atomized by the atomization assembly 300. Then, an aerosol having a relatively consistent flavor can be generated to be used by a user.

The feeding assembly 30 includes a first reel 31a arranged in the first subcavity 10c and a second reel 32a arranged in the second subcavity 10d. The periphery of the first reel 31a is configured to wind the strip-shaped substrate 40. The strip-shaped substrate 40 may be unwound from the first reel 31a, passes through the atomization cavity 10a, and then is wound around the periphery of the second reel 32a. That is, an unatomized strip-shaped substrate 40 is unwound from the first reel 31a, atomized, and then is wound by the second reel 32a.

Illustratively, the two ends of the strip-shaped substrate 40 are wound around the periphery of the first reel 31a and the periphery of the second reel 32a respectively. The second reel 32a rotates to drive the strip-shaped substrate 40 wound around the first reel 31a to pass through the atomization cavity 10a and to be wound around the second reel 32a. The rotation direction of the second reel may be a clockwise direction or an anticlockwise direction.

Unwinding refers to a process that a roll-shaped substrate is radially stripped away, such that the outer layer of the substrate and the inner layer of the substrate are stripped away. The strip-shaped substrate 40 is wound around the periphery of the first reel 31a, and the strip-shaped substrate 40 can be unwound from the first reel 31a. It refers to a process that the outer layer of the strip-shaped substrate 40 and the inner layer of the strip-shaped substrate 40 are stripped away.

Winding refers to a method of winding a continuous product by using a roll, a reel, etc. The strip-shaped substrate 40 passes through the atomization cavity 10a, and then is wound around the periphery of the second reel 32a to be wound by the second reel 32a. It refers to a process that the atomized strip-shaped substrate 40 is wound by using the second reel 32a, that is, wound into a roll.

Specifically, the strip-shaped substrate 40 is a flexible long-strip-shaped structure that has the particular width and thickness, but can be lengthened and bent according to an actual situation.

An aerosol generating substrate for generating an aerosol coats the surface of the strip-shaped substrate 40, permeates the strip-shaped substrate, or is embedded in the strip-shaped substrate. The aerosol generating substrate includes but is not limited to a medicine, a material including nicotine, etc.

Illustratively, the strip-shaped substrate 40 may include a base band and aerosol generating substrates spreading on and coating the base band alternately at intervals.

The base band may be any band that can withstand an atomization temperature, has flexibility, and can be unwound and wound, such as a paper band, a polymer band, a metal base band, and a graphite base band. The base band may alternatively be a metal mesh. The metal mesh may be woven by using a metal wire, or may be formed by providing a plurality of through holes on a metal sheet.

The strip-shaped substrate 40 is wound into a roll, and is mounted to the periphery of the first reel 31a shown in FIG. 3. The end of the strip-shaped substrate 40 is extracted from the roll, passes through the atomization cavity 10a, and then is fixed to the periphery of the second reel 32a. The above operation can be mounted before the atomization substrate assembly 100 leaves a factory, and can be replaced and mounted by a customer. Alternatively, the atomization substrate assembly may be sold independently to be assembled by a user.

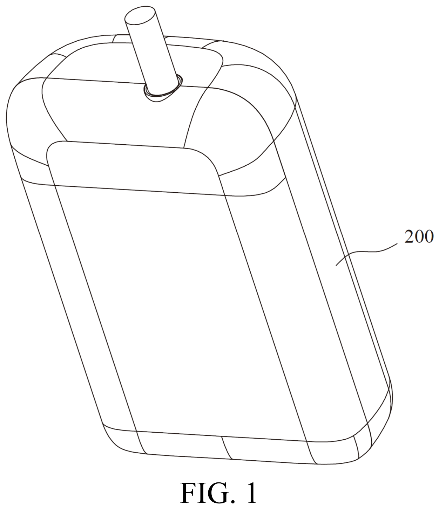

An embodiment of the present disclosure provides an aerosol generating device. With reference to FIG. 1 and FIG. 2, the aerosol generating device includes a main machine 200, an atomization assembly 300, and an atomization substrate assembly in any embodiment of the present disclosure.

The main machine 200 is provided with a mounting space 210a. The atomization substrate assembly 100 is detachably arranged in the mounting space 210a. That is, a new strip-shaped substrate 40 is assembled into the atomization substrate assembly 100, and then the atomization substrate assembly 100 is arranged in the mounting space 210a to be used by a user. After the strip-shaped substrate 40 in the atomization substrate assembly 100 is used, the atomization substrate assembly 100 may be first detached from the mounting space 210a of the main machine 200, and then a new strip-shaped substrate 40 is mounted. Thus, cost of usage by a user is reduced. Or, the entire atomization substrate assembly 100 may be replaced such that convenience of usage by a user can be improved.

Illustratively, with reference to FIG. 2, the main machine 200 includes a main body 210 and an outer cover 220 arranged on the main body 210. The main body 210 includes a mounting space 210a provided with a mounting port. The outer cover 220 is arranged at the mounting port.

To conveniently repair or replace the atomization substrate assembly 100, the atomization substrate assembly 100 is required to be conveniently detached and assembled, and the main body 210 is required to be detachably connected to the outer cover 220. Illustratively, the main body 210 may be connected to the outer cover 220, for example, through a magnetic attraction connection, snap-fitting, a threaded connection, plugging, or ultrasound.

The main machine 200 includes a power supply assembly. The atomization assembly 300 is electrically connected to the power supply assembly and is configured to atomize the strip-shaped substrate 40. Illustratively, at least part of the structure of the atomization assembly 300 extends into the atomization cavity 10a, to atomize the strip-shaped substrate 40 in the atomization cavity 10a. For example, a heating assembly of the atomization assembly 300 extends into the atomization cavity 10a, to atomize the strip-shaped substrate 40 in the atomization cavity 10a.

It should be noted that a specific position of the atomization assembly 300 is not limited herein. Illustratively, in some embodiments, the heating assembly of the atomization assembly 300 may be arranged on the atomization substrate assembly 100. For example, the heating assembly extends into the atomization cavity 10a, and a connection end of the atomization assembly 300 may be arranged on the main machine 200.

In some other embodiments, with reference to FIG. 2, the atomization assembly 300 may alternatively be arranged on the main machine 200. When the atomization substrate assembly 100 is assembled to the mounting space 210a, the heating assembly of the atomization assembly 300 may extend into the atomization cavity 10a.

With reference to FIG. 2, the main machine 200 includes a driving assembly 230. Part of the driving assembly 230 extends into the accommodation cavity 10b and is in drive connection to at least the second reel 32a. That is, the driving assembly 230 can drive the second reel 32a. The driving assembly 230 is arranged on the main machine 200. When the atomization substrate assembly 100 is assembled to the mounting space 210a, the driving assembly 230 is in drive connection to the second reel 32a.

Illustratively, in some embodiments, the driving assembly 230 may be in drive connection to only the second reel 32a. In some other embodiments, the driving assembly 230 may be in drive connection to both the first reel 31a and the second reel 32a.

The driving assembly 230 drives the second reel 32a to rotate, to drive the strip-shaped substrate 40 wound around the first reel 31a to pass through the atomization cavity 10a to be wound around the second reel 32a.

Illustratively, the first reel 31a is a damping rotary shaft. That is, the first reel 31a is subjected to a damping force during rotation. For example, the first reel 31a is connected to a damping gear, or is connected to an electric motor, or is connected to a distortion spring, or is in interference fit with either of the atomization substrate assembly 100 and the main machine 200.

The first reel 31a is arranged to be subjected to a particular damping force during rotation, such that the strip-shaped substrates 40 has a particular pretightening force during rotation. Thus, reliability of the strip-shaped substrates 40 during a movement is improved, a transmission distance of the strip-shaped substrate 40 is more precisely controlled, and the strip-shaped substrate 40 can be prevented from being damaged during the movement.

When user inhalation is required, the second reel 32a is driven by the driving assembly 230 to rotate, to drive the first reel 31a to rotate. Thus, the strip-shaped substrate 40 is continuously unwound from the first reel 31a. The unwound strip-shaped substrate 40 is atomized by the atomization assembly 300 in the atomization cavity 10a along the way. An aerosol generated in the atomization cavity 10a is discharged to be inhaled by a user. The atomized strip-shaped substrate 40 is wound around the second reel 32a. The aerosol generating device may respond to an operation by a user in real time, be started at any time, and continuously supply and atomize a large number of strip-shaped substrates 40, and may further stop supplying and atomizing the strip-shaped substrates at any time almost without a time delay of response time. After a particular time or a particular number of times of user inhalation, this roll of strip-shaped substrate 40 is used up. The user can open the main machine 200 to take out the atomization substrate assembly 100, and directly replace the atomization substrate assembly with a new atomization substrate assembly 100, or open the atomization substrate assembly 100 to replace this roll of strip-shaped substrate with a next roll of strip-shaped substrate 40 by himself/herself.

In the related technology, an unatomized aerosol generating substrate strip and an atomized aerosol generating substrate strip are arranged in the same space. The flavor of the atomized aerosol generating substrate may affect the flavor of the unatomized aerosol generating substrate, such that the flavor of the aerosol generating substrate atomized later is changed to affect user experience.

Moreover, scraps generated by the atomized aerosol generating substrate strip are likely to stick to the unatomized aerosol generating substrate strip. Thus, the aerosol generating substrate strip atomized later generate an odor such as a burnt odor, to affect user experience.

An atomization substrate assembly provided in an embodiment of the present disclosure includes a housing assembly 10, a first partition portion 21, and a feeding assembly 30. The first partition portion 21 is arranged in the housing assembly 10. The first partition portion 21 is configured to partition an accommodation cavity 10b into a first subcavity 10c and a second subcavity 10d that are independent of each other in the housing assembly 10. That is, the first partition portion 21 partitions the space in the housing assembly 10 into two independent spaces, that is, the first subcavity 10c and the second subcavity 10d. However, an unatomized strip-shaped substrate 40 is wound around a first reel 31a in the first subcavity 10c, and an atomized strip-shaped substrate 40 is wound around a second reel 32a in the second subcavity 10d. That is, the unatomized strip-shaped substrate 40 and the atomized strip-shaped substrate 40 are arranged in two independent spaces respectively, such that the unatomized strip-shaped substrate 40 is separated from the atomized strip-shaped substrate 40. The first subcavity 10c is a space in which the unatomized strip-shaped substrate 40 can move, and the second subcavity 10d is a space in which the atomized strip-shaped substrate 40 and foreign objects such as abscissions can move. By arranging the partition structure in the atomization substrate assembly 100 in an embodiment of the present disclosure, a situation that an odor of an unatomized strip-shaped substrate 40 and an odor of an atomized strip-shaped substrate 40 are mixed is reduced to some extent. In addition, scraps generated from the atomized strip-shaped substrate 40 can be prevented from sticking to the unatomized strip-shaped substrate 40. Thus, a burnt odor can be prevented to some extent.

Illustratively, with reference to FIG. 3, the housing assembly 10 is in a flat box shape and includes a box body 11 and a cover body 12 covering the box body 11. The box body 11 abuts against the cover body 12 in the thickness direction. The first partition portion 21 is arranged in the accommodation cavity 10b. The storage space 10e and the accommodation space 10f are defined by the box body 11, the cover body 12, and the first partition portion 21 together. The feeding assembly 30 is arranged at the bottom side of the atomization cavity 10a. The strip-shaped substrate 40 moves in the atomization cavity 10a in the transverse direction. The transverse direction, the thickness direction, and the top-bottom direction are perpendicular to each other.

It should be noted that the first partition portion 21 can partition the accommodation cavity 10b into the first subcavity 10c and the second subcavity 10d that are independent of each other in the housing assembly 10 through a plurality of methods. Illustratively, in some embodiments, with reference to FIG. 3 to FIG. 5, the first subcavity 10c and the second subcavity 10d are arranged at an interval in the plane perpendicular to the thickness direction of the housing assembly 10. That is, the first subcavity 10c and the second subcavity 10d are located in the same plane and are arranged at an interval in the same plane. The first subcavity 10c is separated from the second subcavity 10d by the first partition portion 21.

In some other embodiments, the first subcavity 10c and the second subcavity 10d are arranged at an interval in the thickness direction of the housing assembly 10. That is, the first subcavity 10c and the second subcavity 10d are arranged at an interval in the thickness direction of the housing assembly 10. The first subcavity 10c and the second subcavity 10d are layered in the thickness direction. The first subcavity 10c is separated from the second subcavity 10d by the first partition portion 21.

An embodiment of the present disclosure is described with an example in which the first subcavity 10c and the second subcavity 10d are arranged at an interval in the plane perpendicular to the thickness direction of the housing assembly 10.

Illustratively, the first subcavity 10c includes a storage space 10e for storing the unatomized strip-shaped substrate 40. The second subcavity 10d includes an accommodation space 10f for accommodating the atomized strip-shaped substrate 40. The first reel 31a is arranged in the storage space 10e. The second reel 32a is arranged in the accommodation space 10f. That is, the storage space 10e is configured to store the unatomized strip-shaped substrate 40. The accommodation space 10f is configured to accommodate the atomized strip-shaped substrate 40.

In the related technology, the aerosol generating substrate strip deforms, hardens, gets a rough edge, and is layered after being atomized by an atomization assembly. However, an aerosol generating device in the related technology is not specially configured to accommodate an atomized aerosol generating substrate strip, such that scraps are generated by the atomized aerosol generating substrate strip.

However, in an embodiment of the present disclosure, the accommodation space 10f that is specially configured to accommodate the atomized strip-shaped substrate 40 is arranged in the second subcavity 10d such that scraps can be reduced to some extent. Moreover, the first subcavity 10c is separated from the second subcavity 10d such that less scraps can enter the first subcavity 10c and stick to the unatomized strip-shaped substrate 40. In addition, the second reel 32a is arranged in the accommodation space 10f and is configured to wind the atomized strip-shaped substrate 40. By winding the atomized strip-shaped substrate 40 into a roll, scraps can be further reduced. Moreover, the storage space 10e that is specially configured to store the unatomized strip-shaped substrate 40 is arranged in the first subcavity 10c such that the unatomized strip-shaped substrate 40 can be protected. A probability of an odor of the unatomized strip-shaped substrate 40 is reduced, to make user experience better.

Illustratively, in some embodiments, with reference to FIG. 3 to FIG. 5, the first partition portion 21 has a curved section. The curved section includes a first curved subsection 211 and a second curved subsection 212. The first curved subsection 211 and the second curved subsection 212 are connected to form a roughly S-shaped baffle structure. The curvature center of the first curved subsection 211 and the curvature center of the second curved subsection 212 are located on the opposite sides of the curved section respectively. The first curved subsection 211 forms part of the storage space 10e. The second curved subsection 212 forms part of the accommodation space 10f.

In some other embodiments, the first partition portion 21 may be provided with no curved section. The accommodation cavity 10b is directly divided into the first subcavity 10c and the second subcavity 10d by using a straight segment.

It can be understood that the first partition portion 21 is provided with the curved section including the first curved subsection 211 and the second curved subsection 212. The first curved subsection 211 forms part of the storage space 10e. The second curved subsection 212 forms part of the accommodation space 10f. That is, the storage space 10e and the accommodation space 10f may be set to be in curved shapes, such as cambered shapes. In this way, the strip-shaped substrate 40 can be favorably wound to some extent. The space of the accommodation cavity 10b is fully used such that the size of the atomization substrate assembly 100 can be reduced correspondingly, to make user experience better.

The first curved subsection 211 is connected to the second curved subsection 212. The first curved subsection 211 may be directly connected to the second curved subsection 212, or may be indirectly connected to the second curved subsection. For example, the first partition portion 21 further includes a transition section, and the first curved subsection 211 is connected to the second curved subsection 212 through the transition section. Thus, the storage space 10e and the accommodation space 10f can be more favorably designed.

Illustratively, the first curved subsection 211 is smoothly connected to the second curved subsection 212. For example, the first curved subsection 211 is tangent to the second curved subsection 212 at the connection position. In this way, acute angles or spikes of the storage space 10e and the accommodation space 10f can be reduced, and the strip-shaped substrate 40 can be favorably wound. Moreover, the strip-shaped substrate 40 can be prevented from being scratched or damaged, to improve reliability of the atomization substrate assembly 100. In addition, the space of the accommodation cavity 10b can be fully used.

The first partition portion 21 may include a baffle. The baffle may be integrally formed on the box body 11, and may be formed by the bottom face projecting upwards of the accommodation cavity 10b opposite the opening. The cover body 12 is arranged at the opening, and is attached to the front end face of the baffle, to define the first subcavity 10c and the second subcavity 10d that are independent of each other. Further, in some embodiments, the first partition portion 21 may further include a seal member fitted between the baffle and the cover body 12. The seal member may be made of a seal material such as silicon, to further improve the effect of separating the first subcavity 10c from the second subcavity 10d.

Certainly, in other embodiments, the baffle may be integrally formed on the cover body 12, or partially formed on the box body 11 and partially formed on the cover body 12. In some other embodiments, the baffle may be independently formed and then assembled to the box body 11 or the cover body 12.

Illustratively, with reference to FIG. 3, the feeding assembly 30 includes a storage disk 31 provided with the first reel 31a. The storage disk 31 is rotatably arranged in the storage space 10e. The unatomized strip-shaped substrate 40 is arranged on the storage disk 31. That is, by arranging the storage disk 31, the storage disk 31 may rotate such that the strip-shaped substrate 40 can be favorably unwound from the storage disk 31. Thus, friction between the strip-shaped substrate 40 and the housing assembly 10 in an unwinding process can be reduced.

Certainly, in some other embodiments, the feeding assembly 30 may be provided with no storage disk 31. The unatomized strip-shaped substrate 40 is directly arranged on the housing assembly 10.

Illustratively, with reference to FIG. 3, the feeding assembly 30 includes an accommodation disk 32 provided with the second reel 32a. The accommodation disk 32 is rotatably arranged in the accommodation space 10f. The atomized strip-shaped substrate 40 is arranged on the accommodation disk 32. That is, by arranging the accommodation disk 32, the accommodation disk 32 can rotate such that the strip-shaped substrate 40 can be favorably wound around the accommodation disk 32. Thus, friction between the strip-shaped substrate 40 and the housing assembly 10 in a winding process can be reduced.

Certainly, in some other embodiments, the feeding assembly 30 may be provided with no accommodation disk 32. The atomized strip-shaped substrate 40 is directly arranged on the housing assembly 10.

The strip-shaped substrate 40 deforms, hardens, gets a rough edge and is layered after being atomized by the atomization assembly 300. The size of the strip-shaped substrate 40 is increased in a natural state. In other words, the diameter of the strip-shaped substrate 40 not atomized into a roll is less than the diameter of the strip-shaped substrate having the same length and atomized into a roll. Thus, space for accommodating the atomized strip-shaped substrate 40 is larger than space for storing the unatomized strip-shaped substrate 40. Illustratively, in the plane perpendicular to the thickness direction of the housing assembly 10, the cross-sectional area of the accommodation space 10f is greater than the cross-sectional area of the storage space 10e. In this way, the space of the accommodation space 10f is greater than the space of the storage space 10e, to improve the space utilization rate of the housing assembly 10. The atomization substrate assembly 100 can be favorably miniaturized.

Illustratively, in the plane perpendicular to the thickness direction of the housing assembly 10, the cross-sectional area of the accommodation disk 32 is greater than the cross-sectional area of the storage disk 31. In this way, the space of the accommodation space 10f is greater than the space of the storage space 10e.

Certainly, in other embodiments, the space of the accommodation space 10f may be less than or equal to the space of the storage space 10e. Illustratively, after the strip-shaped substrate 40 penetrates the atomization cavity 10a, by arranging a peeling structure, the atomized aerosol generating substrate is stripped away from the base band. The thickness of the strip-shaped substrate 40 after the peeling is reduced such that the space of the accommodation space 10f can be set to be less than the space of the storage space 10e.

With reference to FIG. 3 to FIG. 5, the curvature center of the first curved subsection 211 and the curvature center of the second curved subsection 212 are located at the opposite sides of the curved section respectively. That is, the first curved subsection 211 and the second curved subsection 212 are curved towards the opposite sides of the curved section respectively. Or the opening of the first curved subsection 211 and the opening of the second curved subsection 212 face the opposite sides of the curved section respectively. For example, the curved section extends in the height direction of the atomization substrate assembly 100. The opening of the first curved subsection 211 and the opening of the second curved subsection 212 face the opposite sides of the atomization substrate assembly 100 in the width direction respectively. In this way, the space of the accommodation cavity 10b can be fully used. The first subcavity 10c and the second subcavity 10d that are independent of each other are obtained through division in a limited space by arranging the curved section. Thus, the size of the atomization substrate assembly 100 is reduced to some extent, to make user experience better.

The curvature center of the first curved subsection 211 and the curvature center of the second curved subsection 212 are set to be located on the opposite sides of the curved section respectively. The accommodation cavity 10b is divided into the first subcavity 10c and the second subcavity 10d by arranging the straight segment instead of the curved section such that the size of the atomization substrate assembly 100 can be reduced.

Illustratively, when projected on the plane perpendicular to the thickness direction of the housing assembly 10, a connection line between the curvature center of the first curved subsection 211 and the curvature center of the second curved subsection 212 coincides with the central axis of the housing assembly 10. That is, when projected on the plane perpendicular to the thickness direction of the housing assembly 10, the curvature center of the first curved subsection 211 and the curvature center of the second curved subsection 212 are located on the projection of the central axis of the housing assembly 10. That is, the first curved subsection 211 and the second curved subsection 212 are both arranged at the central position of the accommodation cavity 10b. For example, the atomization cavity, the accommodation space, and the storage space are linearly arranged in the top-bottom direction. It should be noted that overlapping herein refers to complete overlapping or rough overlapping.

It can be understood that when projected on the plane perpendicular to the thickness direction of the housing assembly 10, the curvature center of the first curved subsection 211 and the curvature center of the second curved subsection 212 are arranged on the central axis. The first curved subsection 211 forms part of the storage space 10e, and the second curved subsection 212 forms part of the accommodation space 10f. In this way, the center of the storage space 10e and the center of the accommodation space 10f can be set on the central axis of the housing assembly 10. The strip-shaped substrate 40 can move in the gap between the first partition portion 21 and the inner wall of the housing assembly 10. Thus, the space of the accommodation cavity 10b can be fully used, and the size of the atomization substrate assembly 100 can be reduced to some extent, to make user experience better.

It can be understood that the strip-shaped substrate 40 wound into a roll is generally cylindrical. The cylindrical strip-shaped substrate 40 is beneficial to unwinding and winding. In this way, the unatomized strip-shaped substrate 40 wound around the first reel 31a may be set to be approximately cylindrical, and the atomized strip-shaped substrate 40 wound around the second reel 32a may be set to be approximately cylindrical. Illustratively, the first curved subsection 211 is a first cambered section, and the second curved subsection 212 is a second cambered section. In this way, the storage space 10e and the accommodation space 10f are also approximately cylindrical. Thus, the space of the accommodation cavity 10b can be fully used such that the strip-shaped substrate 40 can be favorably unwound and wound.

Illustratively, the diameter of the first cambered section is less than the diameter of the second cambered section. In this way, in the plane perpendicular to the thickness direction of the housing assembly 10, the cross-sectional area of the accommodation space 10f can be greater than the cross-sectional area of the storage space 10e. Thus, the space of the accommodation space 10f is greater than the space of the storage space 10e.

The first cambered section is smoothly connected to the second cambered section. In this way, acute angles or spikes of the storage space 10e and the accommodation space 10f can be reduced, and the strip-shaped substrate 40 can be favorably wound. Moreover, the strip-shaped substrate 40 can be prevented from being scratched or damaged, to improve reliability of the atomization substrate assembly 100. In addition, the space of the accommodation cavity 10b can be fully used.

The material inlet 101a and the material outlet 102a are both in clearance fit with the strip-shaped substrate 40 such that the strip-shaped substrate 40 can smoothly move without being stuck. Moreover, the edge of the material inlet 101a and the edge of the material outlet 102a can be prevented from scraping the strip-shaped substrate 40. In some embodiments, the shape of the section (herein referring to the section perpendicular to the traveling direction of the strip-shaped substrate 40 at the material inlet 101a) of the material inlet 101a and/or the material outlet 102a may be the same as or similar to the shape of the section (herein referring to the section perpendicular to the traveling direction of the strip-shaped substrate 40 at the material inlet 101a, or the section in the thickness direction of the strip-shaped substrate 40) of the strip-shaped substrate 40. Illustratively, the section of the strip-shaped substrate 40 is rectangular, the rectangular section has the width size and the thickness size, and the width size is greater than the thickness size. The section of the material inlet 101a and the section of the material outlet 102a are both rectangular or quasi-rectangular, the rectangular or quasi-rectangular section has the width size and the height size, and the width size is greater than the height size. In addition, the width direction of the section of the material inlet 101 a and the width direction of the section of the material outlet 102a are parallel or approximately parallel to the width direction of the section of the strip-shaped substrate 40. The thickness direction of the section of the material inlet 101a and the thickness direction of the section of the material outlet 102a are parallel or approximately parallel to the height direction of the section of the strip-shaped substrate 40. The width size and the height size of the section of the material inlet 101a are greater than the width size and the thickness size of the section of the unatomized strip-shaped substrate 40 respectively. The width size and the height size of the section of the material outlet 102a are greater than the width size and the thickness size of the section of the atomized strip-shaped substrate 40 respectively.

In some embodiments, a gap formed between the periphery of the material inlet 101a and/or the material outlet 102a and the strip-shaped substrate 40 may further be used as an airflow channel for external air to enter the atomization cavity 10a or for an airflow in the atomization cavity 10a to flow out. It can be understood that the size of the gap should not be excessively small. Otherwise draw resistance may be excessively large, and the pressure of the atomization cavity 10a is excessively small to cause inhalation disturbance and affecting inhalation experience. Certainly, the size of the gap should not be excessively large, to reduce leakage of the aerosol generated in the atomization cavity 10a through the material inlet 101a and the outlet 252.

It can be understood that in a case that the sectional size of the material inlet 101a is slightly greater than the sectional size of the strip-shaped substrate 40, the gap formed between the periphery of the material inlet 101a and the strip-shaped substrate 40 is relatively small. In this case, the periphery of the material inlet 101a forms the second partition portion of the at least one partition 20. Thus, it is difficult for the aerosol in the atomization cavity 10a to spill out through the material inlet 101a and pollute a fresh substrate. In some embodiments, the sectional height of the material inlet 101a may be greater than the sectional thickness of the strip-shaped substrate 40 by 0.1 mm to 1.5 mm (including values at the two ends). The sectional width of the material inlet 101a may be greater than the sectional width of the strip-shaped substrate 40 by 0.5 mm to 4 mm (including values at the two ends).

In an embodiment, with reference to FIG. 3 to FIG. 5, the atomization substrate assembly 100 includes an air outlet channel 10n arranged at one end of the housing assembly 10. One end of the air outlet channel 10n is in communication with the atomization cavity 10a, and the other end of the air outlet channel is connected to the outside. The strip-shaped substrate 40 can be atomized by the atomization assembly 300 in the atomization cavity 10a to generate an aerosol. The aerosol enters the air outlet channel 10n from the atomization cavity 10a, and flows out through the air outlet channel 10n for a user to inhale.

Illustratively, in some embodiments, when projected on the plane perpendicular to the thickness direction of the housing assembly 10, a connection line between the curvature center of the first curved subsection 211 and the curvature center of the second curved subsection 212 coincides with the central axis of the housing assembly 10 in the height direction. The air outlet channel 10n is arranged at the top end of the housing assembly 10. That is, the air outlet channel 10n, the atomization cavity 10a, the accommodation space 10f, and the storage space 10e are arranged in the height direction of the atomization substrate assembly 100. In this way, at least the size of the atomization substrate assembly 100 in the width direction can be reduced.

In some other embodiments, when projected on the plane perpendicular to the thickness direction of the housing assembly 10, a connection line between the curvature center of the first curved subsection 211 and the curvature center of the second curved subsection 212 coincides with the central axis of the housing assembly 10 in the width direction. The air outlet channel 10n is arranged at the top end of the housing assembly 10. That is, the accommodation space 10f and the storage space 10e are arranged in the width direction of the atomization substrate assembly 100. In this way, at least the size of the atomization substrate assembly 100 in the height direction can be reduced.

An embodiment of the present disclosure is described with an example in which a connection line between the curvature center of the first curved subsection 211 and the curvature center of the second curved subsection 212 coincides with the central axis of the housing assembly 10 in the height direction. That is, the accommodation space 10f and the storage space 10e are arranged in the height direction of the atomization substrate assembly 100.

Illustratively, with reference to FIG. 3 to FIG. 5, the second curved subsection 212 is arranged near the atomization cavity 10a. The first curved subsection 211 is connected to the end of the second curved subsection 212 away from the atomization cavity 10a. That is, the accommodation space 10f is arranged near the atomization cavity 10a. The storage space 10e is arranged on the side of the accommodation space 10f away from the atomization cavity 10a.

It can be understood that in an attached heating method, to improve the effect of atomizing the strip-shaped substrate 40, the strip-shaped substrate 40 is required to be in close contact with the heating assembly of the atomization assembly 300. By arranging the storage space 10e relatively away from the atomization cavity 10a, the traveling distance of the unwound unatomized strip-shaped substrate 40 in the storage space 10e entering the atomization cavity 10a can be relatively increased. Thus, a traveling trajectory of the unatomized strip-shaped substrate 40 can be adjusted such that the strip-shaped substrate 40 can more flatly travel. Moreover, the strip-shaped substrate is in close contact with the heating assembly such that the unatomized strip-shaped substrate 40 and the heating assembly can be prevented from being damaged. In addition, the accommodation space 10f is arranged at the position relatively near the atomization cavity 10a such that the traveling distance of the atomized strip-shaped substrate 40 entering the accommodation space 10f for winding can be relatively reduced. Scraps generated by the atomized strip-shaped substrate 40 can be reduced to some extent.

Illustratively, with reference to FIG. 3 to FIG. 5, the first curved subsection 211 abuts against the bottom wall of the accommodation cavity 10b. That is, the first subcavity 10c and the second subcavity 10d may be arranged on the opposite sides of the connection position between the first curved subsection 211 and the bottom wall of the accommodation cavity 10b respectively. That is, the first subcavity 10c may be separated from the second subcavity 10d by setting the first curved subsection 211 to abut against the bottom wall of the accommodation cavity 10b.

Illustratively, with reference to FIG. 3 to FIG. 5, the first partition portion 21 is provided with a guide edge 213. The guide edge 213 is smoothly connected to the end of the first curved subsection 211 away from the second curved subsection 212. A traveling channel is defined by the guide edge 213, the curved section, and the inner wall of the housing assembly 10. The strip-shaped substrate 40 is unwound from the first reel 31a, is moved out of the storage space 10e through the traveling channel, and is moved towards the atomization cavity 10a. By setting the guide edge 213 to be smoothly connected to the first curved subsection 211, and defining the traveling channel by the guide edge, the curved section, and the inner wall of the housing assembly 10. The traveling channel forms at least part of the traveling trajectory of the strip-shaped substrate 40.

It should be noted that the guide edge 213 may abut against the side wall of the accommodation cavity 10b or not. In an embodiment of the present disclosure, the guide edge 213 abuts against the side wall of the accommodation cavity 10b. The boundary of the first subcavity 10c is defined at the position where the guide edge 213 abuts against the accommodation cavity 10b.

Illustratively, with reference to FIG. 3 to FIG. 5, the second curved subsection 212 protrudes towards the side facing away from the accommodation space 10f to form a limiting protrusion 212a. The limiting protrusion 212a is configured to limit the strip-shaped substrate 40. That is, the strip-shaped substrate 40 travels through a gap 203a between the limiting protrusion 212a and the housing assembly 10. By arranging the limiting protrusion 212a, the strip-shaped substrate 40 can be limited, and the flatness of the strip-shaped substrate 40 during traveling can be improved.

It can be understood that the limiting protrusion 212a can further form the third partition portion of the at least one partition 20 for preventing the aerosol in the atomization cavity 10a from entering the storage space 10e. The gap 203a between the limiting protrusion 212a and the side wall of the housing assembly 10 forms a substrate pass-through port for the strip-shaped substrate 40 to pass through. The size of the gap 203a is greater than the thickness of the strip-shaped substrate 40 such that the strip-shaped substrate 40 can smoothly pass through the gap 203a and can not be scraped or stuck. Certainly, the size of the gap 203a should not be excessively large. Otherwise, a partition effect will be affected. In some embodiments, the size of the gap 203a may be greater than the thickness of the strip-shaped substrate 40 by 0.2 mm to 3 mm (including values at the two ends).

Illustratively, with reference to FIG. 3 to FIG. 5, the first partition portion 21 is provided with a connection side 214. One end of the connection side 214 is connected to one end of the second curved subsection 212 away from the first curved subsection 211, and the other end of the connection side extends towards the outer side wall of the atomization cavity 10a and is connected to the outer side wall of the atomization cavity 10a. That is, the second curved subsection 212 is connected to the outer side wall of the atomization cavity 10a by the connection side 214, to separate the first subcavity 10c from the second subcavity 10d. In this way, a distance is provided between the accommodation space 10f and the atomization cavity 10a, to provide a mounting space 210a for assembly of other parts of the atomization substrate assembly 100, for example, provide the mounting space 210a for fastening and connection by a fastener.

Certainly, the second curved subsection 212 may alternatively be directly connected to the outer side wall of the atomization cavity 10a, to separate the first subcavity 10c from the second subcavity 10d.

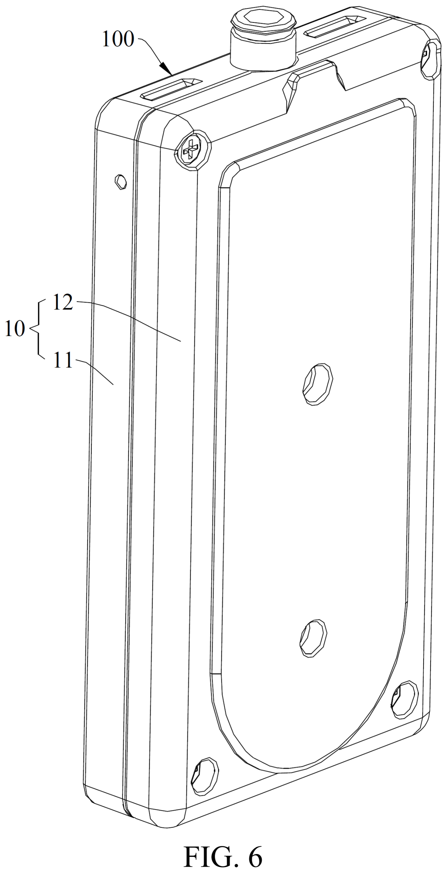

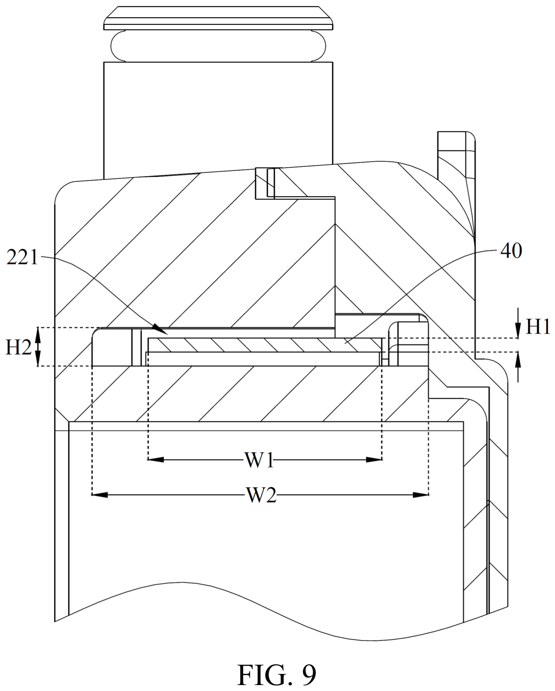

FIG. 6 and FIG. 9 show an atomization substrate assembly 100a according to Embodiment 2 of the present disclosure. The atomization substrate assembly 100 includes a housing assembly 10, and a strip-shaped substrate 40 and a feeding assembly 30 that are accommodated in the housing assembly 10. The structure of the strip-shaped substrate 40 and the structure of the feeding assembly 30 can be obtained with reference to the above descriptions and are not repeated herein.

A storage space 10e, an accommodation space 10f, and an atomization cavity 10a are formed in the housing assembly 10. The storage space 10e is configured to store the unatomized strip-shaped substrate 40 (a fresh substrate). The accommodation space 10f is configured to accommodate the atomized strip-shaped substrate 40 (an atomized substrate). The atomization cavity 10a is configured to heat and atomize the strip-shaped substrate 40 to form an aerosol. The atomization cavity 10a is provided with a material inlet 101a and a material outlet 102a. The strip-shaped substrate 40 can enter the atomization cavity 10a through the material inlet 101a, is heated and atomized in the atomization cavity 10a, and then is transferred out through the material outlet 102a.

In the housing assembly 10, at least one partition 20 may be formed between the storage space 10e and the accommodation space 10f, and at least one partition may be formed between the storage space and the atomization cavity 10a. Thus, an aerosol overflowing from the atomization cavity 10a can be prevented from entering the storage space 10e and causing pollution, and a tobacco residue scrap or an odor generated in the accommodation space 10f can be prevented from entering the storage space 10e and polluting the fresh substrate.

In the embodiment, the at least one partition 20 includes a first partition portion 21 between the storage space 10e and the accommodation space 10f, a second partition portion 22 between the storage space 10e and the atomization cavity 10a, and a third partition portion 23 between the storage space 10e and the second partition portion 22.

The first partition portion 21 separates the storage space 10e from the accommodation space 10f. On one hand, a tobacco residue scrap or an odor in the accommodation space 10f or a nearby region can be prevented from entering the storage space 10e and causing pollution. On the other hand, an aerosol overflowing from the material outlet 102a of the atomization cavity 10a in the direction of the accommodation space 10f can be prevented from entering the storage space 10e. The specific structure of the first partition portion 21 can be obtained with reference to the descriptions in Embodiment 1 and is not repeated herein.

The second partition portion 22 may be arranged near the material inlet 101a or at the material inlet 101a. An objective of the second partition portion 22 is to prevent, to a maximum extent, the aerosol in the atomization cavity 10a from permeating the storage space 10e and polluting the fresh substrate. In the embodiment, the second partition portion 22 is arranged at the material inlet 101a, and may include a main rib, a reinforcing rib, a block, a baffle, etc. that protrudes from at least part of the edge of the material inlet 101a. The second partition portion 22 may be integrally formed on the box body 11 and/or the cover body 12. Certainly, in other embodiments, the second partition portion 22 may alternatively include an independently arranged partition member such as a seal member, a baffle, or a one-way valve. For example, the second partition portion 22 may alternatively include a silicon seal member. The silicon seal member may be mounted at the material inlet 101a or near the material inlet 101a to implement a partition function. For another example, a one-way feed valve is arranged at the material inlet 101a, to prevent the aerosol from permeating the outside of the material inlet 101a.

A substrate pass-through port 221 for the strip-shaped substrate 40 to pass through is formed on the second partition portion 22. The substrate pass-through port 221 is in clearance fit with the strip-shaped substrate 40, to ensure that the strip-shaped substrate 40 normally runs without being scraped. In some embodiments, the shape of the section (herein referring to the section perpendicular to the traveling direction of the strip-shaped substrate 40 at the substrate pass-through port 221) of the substrate pass-through port 221 may be the same as or similar to the shape of the section (herein referring to the section perpendicular to the traveling direction of the strip-shaped substrate 40 at the substrate pass-through port 221, or the section in the thickness direction of the strip-shaped substrate 40) of the strip-shaped substrate 40. Thus, a partition effect can be improved. Illustratively, the section of the strip-shaped substrate 40 is rectangular. The section of the substrate pass-through port 221 is rectangular or quasi-rectangular. The height H2 of the substrate pass-through port 221 is greater than the thickness H1 of the strip-shaped substrate 40. The width W2 of the substrate pass-through port 221 is greater than the width W1 of the strip-shaped substrate 40.

Certainly, a gap between the substrate pass-through port 221 and the strip-shaped substrate 40 should not be excessively large. Otherwise, a partition effect will be affected.

The third partition portion 23 is arranged between the second partition portion 22 and the storage space 10e, to perform a multi-protection function. Thus, an aerosol permeating the storage space 10e can be further reduced. The third partition portion 23 may include a main rib, a reinforcing rib, a block, a baffle, etc. that is integrally formed on the box body 11 and/or the cover body 12, or may include a partition member such as an independently formed seal member or a baffle.

A substrate pass-through port 231 for the strip-shaped substrate 40 to pass through is formed on the third partition portion 23. The substrate pass-through port 231 is in clearance fit with the strip-shaped substrate 40, to ensure that the strip-shaped substrate 40 normally runs without being scraped. In some embodiments, the shape of the section (herein referring to the section perpendicular to the traveling direction of the strip-shaped substrate 40 at the substrate pass-through port 231) of the substrate pass-through port 231 may be the same as or similar to the shape of the section (herein referring to the section perpendicular to the traveling direction of the strip-shaped substrate 40 at the substrate pass-through port 231, or the section in the thickness direction of the strip-shaped substrate 40) of the strip-shaped substrate 40. Thus, a partition effect can be improved. Illustratively, the section of the strip-shaped substrate 40 is rectangular. The section of the substrate pass-through port 231 is rectangular or quasi-rectangular. The height H3 of the substrate pass-through port 231 is greater than the thickness H1 of the strip-shaped substrate 40. The width of the substrate pass-through port 231 is greater than the width of the strip-shaped substrate 40.

Certainly, a gap between the substrate pass-through port 231 and the strip-shaped substrate 40 should not be excessively large. Otherwise, a partition effect will be affected.

It can be understood that the at least one partition 20 may be a combination of the first partition portion 21 between the storage space 10e and the accommodation space 10f, the second partition portion 22 between the storage space 10e and the atomization cavity 10a, and the third partition portion 23 between the storage space 10e and the second partition portion 22. The numbers and positions of the three partition portions are not limited. The at least one partition may alternatively not limited to the above combination.

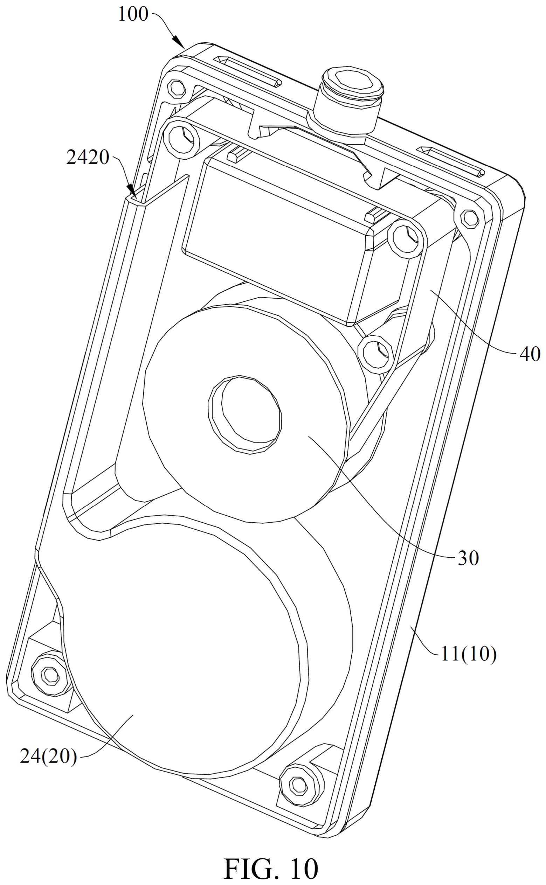

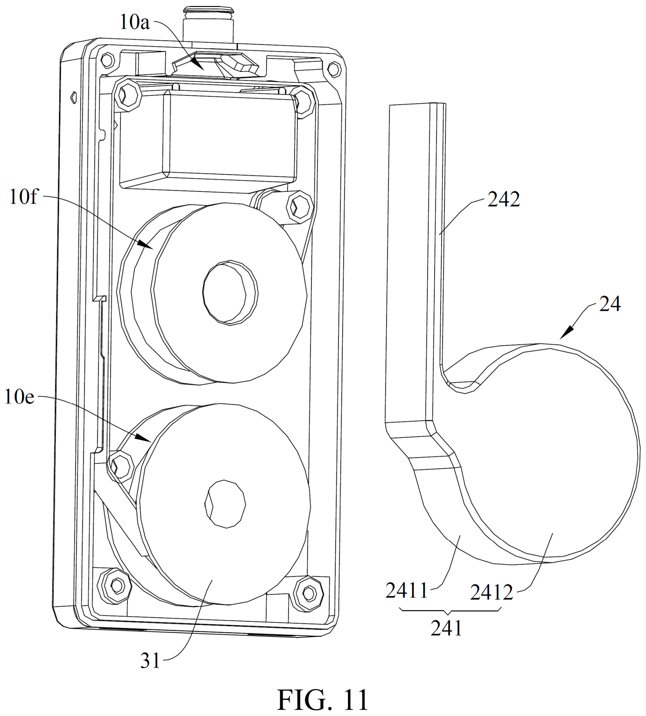

FIG. 10 and FIG. 11 show an atomization substrate assembly 100 according to Embodiment 3 of the present disclosure. The atomization substrate assembly 100 includes a housing assembly 10, a strip-shaped substrate 40, an isolating cover 24, and a feeding assembly 30 that are accommodated in the housing assembly 10. The structure of the housing assembly 10, the structure of the strip-shaped substrate 40 and the structure of the feeding assembly 30 can be obtained with reference to the above descriptions and are not repeated herein.

The isolating cover 24 covers at least the unatomized strip-shaped substrate 40 (the fresh substrate) stored in the storage space 10e. The fresh substrate is arranged in a relatively sealed space, to be protected to the greatest extent.

In some embodiments, the isolating cover 24 may include a cover body 241 for covering the fresh substrate in the storage space 10e and an extending portion 242 extending outwards from the cover body 241. The cover body 241 covers the storage disk 31, and may include an end cover 2412 and an annular side wall 2411 surrounding at least part of the periphery of the end cover 2412. The inner diameter of the annular side wall 2411 is greater than the outer diameter of the storage disk 31, to ensure that the storage disk 31 can smoothly rotate in the cover body 241. The peripheral side of the annular side wall 2411 is provided with an opening for the strip-shaped substrate 40 to pass through.

The extending portion 242 extends from the opening of the annular side wall 2411. The extending direction of the extending portion 242 is along the traveling direction of the strip-shaped substrate 40. The extending portion 242 covers at least part of the strip-shaped substrate 40 between the storage space 10e and the atomization cavity 10a. Preferably, the far end (the end away from the cover body 241) of the extending portion 242 may extend towards the material inlet 101a as far as possible. The far end of the extending portion 242 is near the material inlet 101a as far as possible, to protect the fresh substrate in a larger range.

A channel 2420 for the strip-shaped substrate 40 to pass through is formed in the extending portion 242. The channel 2420 may be in clearance fit with the strip-shaped substrate 40, to ensure smooth running of the strip-shaped substrate 40. In some embodiments, the shape of the section (herein referring to the section perpendicular to the traveling direction of the strip-shaped substrate 40 in the channel 2420) of the channel 2420 may be the same as or similar to the shape of the section (herein referring to the section perpendicular to the traveling direction of the strip-shaped substrate 40 in the channel 2420, or the section in the thickness direction of the strip-shaped substrate 40) of the strip-shaped substrate 40. Thus, a partition effect can be improved. Illustratively, the section of the strip-shaped substrate 40 is rectangular. The section of the channel 2420 is rectangular or quasi-rectangular. The height of the channel 2420 is greater than the thickness of the strip-shaped substrate 40. The width of the channel 2420 is greater than the width of the strip-shaped substrate 40.

In the embodiment, the isolating cover 24 is an independently formed component. The material of the isolating cover 24 includes but is not limited to silicon, plastic, metal, etc. After the strip-shaped substrate 40 is assembled to the box body 11, the isolating cover 24 covers the box body 11, to form a relatively sealed space together with the box body 11 to protect a fresh substrate.

It can be understood that in other embodiments, any deformation can be made on the structure of the isolating cover 24 according to requirements. For example, the isolating cover 24 may include no cover body 241. The feature of the cover body 241 may be implemented by the cover body 12. That is, the box body 11, the annular side wall 2411, and the cover body 12 together form a relatively sealed space to seal the storage space 10e. In some other embodiments, all or part of the structure of the isolating cover 24 may be integrally formed on the box body 11 or the cover body 12. For example, the extending portion 242 and/or the annular side wall 2411 are integrally formed on the box body 11.

In the descriptions of the present disclosure, the descriptions of a reference term such as "an embodiment," "some embodiments," "an example," "a particular example," or "some examples" means that a particular feature, structure, material, or characteristic that is described in combination with the embodiment or the example is included in at least one embodiment or example of the embodiments of the present disclosure. In the present disclosure, the schematic expressions of the above terms are not certainly directed at the same embodiments or examples. Moreover, the particular feature, structure, material or characteristic described can be combined in any suitable manner in any one or more embodiments or examples. In addition, without mutual contradiction, a person skilled in the art can combine different embodiments or examples described in the present disclosure, as well as features in the different embodiments or examples.

The above embodiments are merely preferred embodiments of the present disclosure, but are not used for limiting the present disclosure. A person skilled in the art can make various alterations and variations to the present disclosure. Any modifications, equivalent substitutions, improvements, etc. made within the spirit and principles of the present disclosure shall fall within the scope of protection of the present disclosure.

## Claims

1. An atomization substrate assembly, comprising:
a housing assembly, wherein
the housing assembly is provided with a storage space for storing an unatomized strip-shaped substrate, an atomization cavity for heating and atomizing the strip-shaped substrate to form an aerosol, and an accommodation space for accommodating the atomized strip-shaped substrate; and
at least one partition is formed between the storage space and the accommodation space, and/or at least one partition is formed between the storage space and the atomization cavity.

2. The atomization substrate assembly of claim 1, wherein the at least one partition comprises a first partition portion separating the storage space from the accommodation space.

3. The atomization substrate assembly of claim 2, wherein the first partition portion comprises a first curved subsection and a second curved subsection connected to the first curved subsection, the first curved subsection forms part of the storage space, and the second curved subsection forms part of the accommodation space.

4. The atomization substrate assembly of claim 3, wherein the first curved subsection and the second curved subsection are connected to form an S-shaped baffle structure.

5. The atomization substrate assembly of claim 1, wherein the atomization cavity is provided with a material inlet for the strip-shaped substrate to enter the atomization cavity, and
the at least one partition comprises a second partition portion arranged near or at the material inlet.

6. The atomization substrate assembly of claim 5, wherein a first substrate pass-through port for the strip-shaped substrate to pass through is formed on the second partition portion, and the first substrate pass-through port is in clearance fit with the strip-shaped substrate.

7. The atomization substrate assembly of claim 6, wherein in the thickness direction of the strip-shaped substrate, the shape of the section of the first substrate pass-through port is the same as or similar to the shape of the section of the strip-shaped substrate.

8. The atomization substrate assembly of claim 5, wherein the at least one partition comprises a third partition portion arranged between the second partition portion and the storage space.

9. The atomization substrate assembly of claim 8, wherein a second substrate pass-through port for the strip-shaped substrate to pass through is formed on the third partition portion, and the second substrate pass-through port is in clearance fit with the strip-shaped substrate.

10. The atomization substrate assembly of claim 9, wherein in the thickness direction of the strip-shaped substrate, the shape of the section of the second substrate pass-through port is the same as or similar to the shape of the section of the strip-shaped substrate.

11. The atomization substrate assembly of claim 5, wherein the material inlet is in clearance fit with the strip-shaped substrate.

12. The atomization substrate assembly of claim 11, wherein in the thickness direction of the strip-shaped substrate, the shape of the section of the material inlet is the same as or similar to the shape of the section of the strip-shaped substrate.

13. The atomization substrate assembly of claim 1, wherein the at least one partition comprises an isolating cover, and the isolating cover covers at least the strip-shaped substrate stored in the storage space.

14. The atomization substrate assembly of claim 13, wherein the isolating cover comprises:
a cover body, wherein the cover body covers the strip-shaped substrate stored in the storage space; and
an extending portion, wherein one end of the extending portion is connected to the cover body, the other end of the extending portion extends in the direction of the atomization cavity, and the extending portion covers at least part of the strip-shaped substrate between the storage space and the atomization cavity.

15. The atomization substrate assembly of claim 14, wherein a channel for the strip-shaped substrate to pass through is formed in the extending portion, and the channel is in clearance fit with the strip-shaped substrate.

16. The atomization substrate assembly of claim 15, wherein in the thickness direction of the strip-shaped substrate, the shape of the section of the channel is the same as or similar to the shape of the section of the strip-shaped substrate.

17. The atomization substrate assembly of any one of claims 1 to 16, wherein the at least one partition comprises a partition structure integrally formed on the housing assembly, and/or a partition member that is formed independently and then is mounted to the housing assembly.

18. An aerosol generating device, comprising:
a main machine, wherein the main machine is provided with a mounting space; and
the atomization substrate assembly of any one of claims 1 to 17, wherein the atomization substrate assembly is arranged in the mounting space.
